## Europäisches Patentamt

## European Patent Office

## Office européen des brevets

(11) Veröffentlichungsnummer: **0 445 602 B1**

(12) # EUROPÄISCHE PATENTSCHRIFT

(45) Veröffentlichungstag der Patentschrift: **03.05.95**

(51) Int. Cl.[6]: **C07C 263/20, C07C 265/14**

(21) Anmeldenummer: **91102593.0**

(22) Anmeldetag: **22.02.91**

(54) **Verfahren zur Herstellung von Mischungen aus Diphenylmethan-diisocyanaten und Polyphenyl-polymethylen-polyisocyanaten mit einer verminderten Iodfarbzahl.**

(30) Priorität: **06.03.90 DE 4006976**

(43) Veröffentlichungstag der Anmeldung:
**11.09.91 Patentblatt 91/37**

(45) Bekanntmachung des Hinweises auf die Patenterteilung:
**03.05.95 Patentblatt 95/18**

(84) Benannte Vertragsstaaten:
**AT BE CH DE DK ES FR GB IT LI NL SE**

(56) Entgegenhaltungen:
**DE-A- 2 804 375**
**GB-A- 1 014 043**
**GB-A- 1 042 858**
**US-A- 3 644 457**

(73) Patentinhaber: **BASF Aktiengesellschaft**
**Carl-Bosch-Strasse 38**
**D-67063 Ludwigshafen (DE)**

(72) Erfinder: **Scherzer, Dietrich, Dr.**
**Prager Strasse 33**
**W-6700 Ludwigshafen (DE)**
Erfinder: **Minges, Roland, Dr.**
**Auf der Setz 23**
**W-6718 Gruenstadt (DE)**

Erfinder: **Langer, Werner, Dr.**
**Wittelsbachstrasse 41**
**W-6700 Ludwigshafen (DE)**
Erfinder: **Bruchmann, Bernd, Dr.**
**Giselherstrasse 79**
**W-6700 Ludwigshafen (DE)**
Erfinder: **Heider, Wolfgang**
**Albert-Einstein-Allee 16**
**W-6703 Limburgerhof (DE)**
Erfinder: **Keller, Peter, Dr.**
**Brunnengasse 24**
**W-6940 Weinheim (DE)**
Erfinder: **Schmitt, Arnold**
**Burgunderstrasse 13**
**W-6701 Friedelsheim (DE)**
Erfinder: **Van Pee, Willy, Dr.**
**Bunderbeeklaan 18**
**B-2080 Kapellen (BE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Erfindungsgegenstand ist ein Verfahren zur Herstellung von Mischungen aus Diphenylmethan-diisocyanaten und Polyphenyl-polymethylen-polyisocyanaten, sogenanntes Roh-MDI, mit einer reduzierten Iodfarbzahl durch Umsetzung der entsprechenden Mischungen aus Diphenylmethan-diaminen und Polyphenyl-polymethylen-polyaminen, sogenanntes Roh-MDA, mit Phosgen in Gegenwart mindestens eines inerten organischen Lösungsmittels, wobei man der Reaktionsmischung nach beendeter Phosgenierung niedermolekulare Alkanole oder mehrwertige Alkohole oder Mischungen davon in einer wirksamen Menge einverleibt.

Roh-MDI, einer der technisch bedeutendsten Ausgangsstoffe zur Herstellung von Polyisocyanat-polyadditionsprodukten, beispielsweise Urethan- oder Urethan- und Isocyanuratgruppen enthaltenden Schaumstoffen, und von 4,4'-Diphenylmethan-diisocyanat, einer wichtigen Aufbaukomponente zur Herstellung von Polyurethan(PU)-Elastomeren, -Fasern, -Dichtungsmassen, -Klebstoffen u.a. wird bekanntermaßen hergestellt durch Phosgenierung von Roh-MDA, üblicherweise in Gegenwart eines inerten organischen Lösungsmittels. Roh-MDA wird seinerseits erhalten durch Kondensation von Anilin und Formaldehyd in Gegenwart von sauren Katalysatoren, wobei in Abhängigkeit von den gewählten Mengenverhältnissen der Ausgangsstoffe und den Reaktionsbedingungen sowie den unterschiedlichen Verfahren der prozentuale Anteil an Diphenylmethan-diaminen und den homologen Polyphenyl-polymethylen-polyaminen sowie ihren Isomeren gesteuert werden kann (Kunststoff-Handbuch, Band 7, Polyurethane, 1. Auflage 1966 und 2. Auflage 1983, Carl-Hanser-Verlag, München, Wien). Wird die Kondensation von Anilin und Formaldehyd z.B. in Gegenwart von schwach sauren Katalysatoren durchgeführt, so erhält man Roh-MDA-Gemische mit einem relativ hohen Anteil an 2,2'- und 2,4'-Diamino-diphenylmethanen, während Roh-MDA-Gemische mit einem großen Gehalt an 4,4'-Diamino-diphenylmethan und gleichzeitig geringem Anteil an 2,4'-Diamino-diphenylmethan nur in Gegenwart von größeren Mengen stark saurer Katalysatoren, vorzugsweise von starken Mineralsäuren, wie z.B. Salzsäure, hergestellt werden können.

Das Verhältnis von Diamino-diphenylmethan-Isomeren zu den höheren Homologen im Roh-MDA ist ferner abhängig vom Anilin-Formaldehyd-Verhältnis und der Kondensationstemperatur, wobei größere Anilin-Formaldehyd-Verhältnisse und niedrige Kondensationstemperaturen hohe Diamino-diphenylmethangehalte ergeben (CA-A-700 026).

Nachteilig an diesen Herstellungsverfahren, die in einer Vielzahl von Literatur- und Patentpublikationen beschrieben werden, ist die Bildung von mehr oder weniger stark gefärbten Roh-MDA, deren Farbe von schwarz über dunklere und hellere Brauntöne bis zu ocker variieren kann. Nachteilig ist ferner, daß diese Verfärbungen auch durch die anschließende Phosgenierung zur Herstellung der entsprechenden Roh-MDI nicht oder nur unzureichend vermindert werden und das gebildete Roh-MDI nicht durch Destillation gereinigt werden kann. Diese unerwünschte Verfärbung wird außerdem in den Folgeprodukten wirksam, so daß auch die aus gefärbtem Roh-MDI hergestellten gegebenenfalls zellhaltigen Polyisocyanat-Polyadditionsprodukte nicht farblos sind. Obgleich die Eigenfarbe der Polyisocyanat-Polyadditionsproduktederen mechanische Eigenschaften nicht negativ beeinflußt, werden vom Verbraucher im wesentlichen farblose Produkte gewünscht.

Es hat daher nicht an Versuchen gefehlt, die Verfärbungen von Roh-MDI zu vermindern und die hergestellten Polyisocyanate durch geeignete Verfahrensmaßnahmen oder Zusatzstoffe zu stabilisieren.

Nach Angaben der US-A-2 885 420 können organische Polyisocyanate gegen eine Verfärbung durch die Zugabe von 0,01 bis 0,5 Gew.-%, bezogen auf das Polyisocyanatgewicht, eines aromatischen, cycloaliphatischen oder aliphatischen Ethers oder Thioethers stabilisiert werden.

Zur Beseitigung von als Gelbildungskatalysatoren wirkenden Verunreinigungen in organischen Diisocyanatlösungen, werden diesen gemäß DE-A-1 280 855 (GB 1 097 219) etwa 0,001 bis 1 Gew.-%, bezogen auf das Gewicht des Diisocyanats, an Phosphorsäure zugesetzt.

Die GB-B-1 465 014 beschreibt den Zusatz von Glycidol in einer Menge von 0,001 bis 0,25 Gew.-%, bezogen auf das Diisocyanatgewicht, zur Verbesserung der Lagerstabilität von destillierten Diphenylmethandiisocyanaten.

Die EP-B-0 183 976 (US-A-4 677 221) betrifft ein Verfahren zur Herstellung von wärmefarbbeständigen (cyclo)aliphatischen Diisocyanaten, wobei man technisches Diisocyanat mit aliphatisch und/oder cycloaliphatisch gebundenen Isocyanatgruppen in Gegenwart von 0,1 bis 3 Gew.-% einer in dem Diisocyanat löslichen Verbindung, welche mindestens 3 Gew.-% an Struktureinheiten der Formel -NH-CO- aufweist, während eines Zeitraums von bis zu 5 Stunden auf eine Temperatur von 100 bis 220°C erhitzt und anschließend das so behandelte Diisocyanat durch Destillation reinigt. Das Verfahren ist nicht auf die Behandlung von Roh-MDI übertragbar, da, wie bereits ausgeführt wurde, dieses nicht destillierbar ist.

Nach Angabe der US-A-4 465 639 werden Roh-MDI nach beendeter Phosgenierung, aber vor der vollständigen Abtrennung des Phosgens, 0,1 bis 5 Gew.-% Wasser, bezogen auf das Polyisocyanatgewicht der Reaktionsmischung, einverleibt. Durch diese Maßnahme kann die Farbe des Roh-MDI und der daraus hergestellten PU-Schaumstoffe aufgehellt werden. Ferner wird der Anteil an höhermolekularen MDI-Homologen im Roh-MDI beträchtlich erniedrigt und die Viskosität reduziert. Obgleich auf diese Weise die Jodfarbzahl des Roh-MDI gesenkt werden kann, sind mit dieser Methode auch erhebliche Nachteile verbunden. Durch die Gegenwart von Wasser wird die korrodierende Wirkung der Chlor, Chlorwasserstoff und Phosgen enthaltenden Reaktionsmischung auf die Apparate der Produktionsanlage beträchtlich verstärkt und dadurch das Leckagerisiko, verbunden mit einem Ausbruch von toxischem Phosgen oder einer phosgenhaltigen Reaktionsmischung, erhöht. Aus Sicherheitsgründen wird daher Feuchtigkeit in jeder Form bei der Phosgenierung zweckmäßigerweise im wesentlichen vollständig ausgeschlossen.

Die Aufgabe der vorliegenden Erfindung bestand darin unter Vermeidung der genannten Nachteile die Iodfarbzahl von Roh-MDI dem durch Wasserzugabe erreichbare Niveau anzugleichen oder noch stärker zu vermindern, wobei insbesondere auf den Zusatz von Wasser verzichtet werden sollte.

Diese Aufgabe konnte überraschenderweise gelöst werden durch den Zusatz von ein- oder/und mehrwertigen Alkoholen zu der phosgenhaltigen Reaktionsmischung nach beendeter Phosgenierung.

Gegenstand der Erfindung ist somit ein Verfahren zur Herstellung von Roh-MDI mit einer reduzierten Iodfarbzahl durch Umsetzung der entsprechenden Roh-MDA mit Phosgen in Gegenwart mindestens eines inerten organischen Lösungsmittels bei erhöhter Temperatur,
nach beendeter Phosgenierung Abtrennung des überschüssigen Phosgens und Lösungsmittels und
thermischer Behandlung des erhaltenen Reaktionsprodukts,
das dadurch gekennzeichnet ist, daß man der Reaktionsmischung nach beendeter Phosgenierung niedermolekulare Alkanole oder vorzugsweise mehrwertige Alkohole oder Mischungen aus niedermolekularen Alkanolen und mehrwertigen Alkoholen in einer wirksamen Menge einverleibt.

Durch den erfindungsgemäß angewandten Zusatz der ein- und/oder mehrwertigen Alkohole kann die Iodfarbzahl des Roh-MDI beträchtlich gesenkt werden, z.B. auf Werte von kleiner als 60, vorzugsweise von 40 bis 6 und kleiner und insbesondere von 20 bis 6 und kleiner.

Die nach dem erfindungsgemäßen Verfahren hergestellten Mischungen aus Diphenylmethan-diisocyanaten (MDI) und Polyphenyl-polymethylen-polyisocyanaten besitzen ferner vorteilhafterweise einen MDI-Isomerengehalt von 30 bis 90 Gew.-%, vorzugsweise von 30 bis 70 Gew.-%, einen NCO-Gehalt von 31±2 Gew.-%, vorzugsweise von 31±1,0 Gew.-%, jeweils bezogen auf das Roh-MDI-Gewicht, und eine Viskosität von maximal 2000 mPa•s, vorzugsweise von 50 bis 500 mPa•s, gemessen bei 23°C.

Roh-MDI mit solchen Isomeren- und Homologenzusammensetzungen können, wie bereits ausgeführt wurde, durch Phosgenierung von Roh-MDA mit entsprechenden Produktzusammensetzungen in Gegenwart mindestens eines inerten organischen Lösungsmittels nach bekannten Verfahren hergestellt werden.

Geeignete Roh-MDA werden vorteilhafterweise erhalten durch Kondensation von Anilin und Formaldehyd in einem Molverhältnis von 6 bis 1,6 : 1, vorzugsweise von 3 bis 2 : 1 und einem Molverhältnis von Anilin zu sauren Katalysatoren von 1 : 0,98 bis 0,01, vorzugsweise 1 : 0,8 bis 0,2.

Der Formaldehyd wird vorzugsweise in Form einer wäßrigen Lösung, z.B. als handelsübliche 30 bis 40 gew.-%ige Lösung, verwendet.

Als saure Katalysatoren haben sich Protonendonatoren, wie z.B. saure Ionenaustauscherharze oder starke organische und vorzugsweise anorganische Säuren bewährt. Als starke Säuren sind hierbei solche mit einem pKs-Wert kleiner als 1,5 - bei mehrbasischen Säuren gilt dieser Wert für die erste Wasserstoffdissoziation - zu verstehen. Beispielhaft genannt seien Salzsäure, Schwefelsäure, Phosphorsäure, Fluorsulfonsäure und Oxalsäure. Chlorwasserstoff kann auch gasförmig eingesetzt werden. Vorzugsweise zur Anwendung kommt wäßrige Salzsäure in Konzentrationen von etwa 25 bis 31 Gew.-%.

In Betracht kommende Verfahren zur Roh-MDA-Herstellung werden beispielsweise beschrieben in CA-A-700 026, DE-B-22 27 110 (US-A-4 025 557), DE-B-22 38 920 (US-A-3 996 283), DE-B-24 26 116 (GB-A-1 450 632), DE-A-12 42 623 (US-A-3 478 099), GB-A-1 064 559 und DE-A-32 25 125.

Als andere Ausgangskomponente zur Herstellung von Roh-MDI wird Phosgen verwendet. Das gasförmige Phosgen kann als solches oder in Verdünnung mit unter den Reaktionsbedingungen inerten Gasen, wie Stickstoff, Kohlenmonoxid u.a. eingesetzt werden. Das Molverhältnis von Roh-MDA zu Phosgen wird zweckmäßigerweise so bemessen, daß pro $NH_2$-Gruppe 1 bis 10 Mol, vorzugsweise 1,3 bis 4 Mol, Phosgen in der Reaktionsmischung vorliegen.

Als inerte organische Lösungsmittel kommen Verbindungen in Betracht, in welchen das Roh-MDA und das Phosgen mindestens teilweise löslich sind.

Als Lösungsmittel vorzüglich bewährt haben sich chlorierte, aromatische Kohlenwasserstoffe, wie z.B. o-Dichlorbenzol, p-Dichlorbenzol, Trichlorbenzole, die entsprechenden Toluole und Xylole, Chlorethylbenzol, Monochlordiphenyl, $\alpha$- bzw. $\beta$-Naphthylchlorid und insbesondere Chlorbenzol und Phthalsäuredialkylester, wie Diethylphthalat. Die Lösungsmittel können einzeln oder als Gemische verwendet werden. Zweckmäßigerweise wird ein Lösungsmittel verwendet, das einen niedrigeren Siedepunkt besitzt als die MDI-Isomeren, damit das Lösungsmittel leicht durch Destillation vom Roh-MDI abgetrennt werden kann. Die Menge an Lösungsmittel wird zweckmäßig so bemessen, daß die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist.

Das Roh-MDA kann als solches oder gelöst in organischen Lösungsmitteln zur Anwendung kommen. Insbesondere verwendet man jedoch Roh-MDA-Lösungen mit einem Amingehalt von 2 bis 40 Gew.-%, vorzugsweise von 5 bis 20 Gew.-%, bezogen auf das Gesamtgewicht der Aminlösung.

Zur Verminderung der Iodfarbzahl werden der phosgenhaltigen Reaktionsmischung erfindungsgemäß niedermolekulare Alkanole oder vorzugsweise mehrwertige Alkohole oder Mischungen aus niedermolekularen Alkanolen und mehrwertigen Alkoholen einverleibt. Als mehrwertige Alkohole geeignet sind ferner Stärke, wie z.B. Weizen-, Mais-, Reis- oder Kartoffelstärke und Zuckerderivate.

Als niedermolekulare Alkanole erfindungsgemäß verwendbar sind sekundäre, tertiäre und vorzugsweise primäre Alkanole mit verzweigtkettigen oder vorzugsweise linearen Alkylresten mit 1 bis 10, vorzugsweise 1 bis 4 Kohlenstoffatomen. Beispielhaft genannt seien Methanol, Ethanol, n- und iso-Propanol, n-Butanol, sek.-Butanol und tert.-Butanol, Pentanol, Hexanol, n- und iso-Octanole.

Geeignete mehrwertige Alkohole sind zweckmäßigerweise 2- bis 8-wertig, vorzugsweise 2- oder 3-wertig und besitzen, ausgenommen die höhermolekulare Stärke, zweckmäßigerweise ein Molekulargewicht von 60 bis 350, vorzugsweise von 60 bis 150. Als mehrwertige Alkohole kommen beispielsweise in Betracht: Alkandiole, vorteilhafterweise solche mit 2 bis 8, vorzugsweise 2 bis 6 Kohlenstoffatomen wie z.B. 1,3-Propandiol, 2,2-Dimethyl-propandiol-1,3, 1,4-Butandiol, 2-Methyl-, 2,2-Dimethyl-butandiol-1,4, 1,5-pentandiol, 2-Ethyl-pentandiol, 1,6-Hexandiol und vorzugsweise Ethandiol, dreiwertige Alkohole mit vorteilhafterweise 3 bis 9 Kohlenstoffatomen, wie z.B. Trimethylolpropan, Triethanolpropan, Hexantriole, Trihydroxycyclohexan und vorzugsweise Glycerine, 4- und höherwertige Alkohole mit vorteilhafterweise 4 bis 12 Kohlenstoffatomen, wie z.B. Hexantetrole, Pentaerythrit, Sorbit, Inosit und Saccharose. Von den ein- und mehrwertigen Alkoholen besonders bewährt hat sich Glycerin, so daß dieser dreiwertige, technisch leicht zugängliche Alkohol insbesondere eingesetzt wird.

Die niedermolekularen Alkanole und/oder mehrwertigen Alkohole können in reiner oder technischer Qualität verwendet werden, wobei jedoch der Wassergehalt der technischen Produkte möglichst gering, zweckmäßigerweise unter 0,1 Gew.-% betragen sollte. Verwendbar sind ferner Mischungen aus Alkanolen oder mehrwertigen Alkoholen oder Mischungen aus mindestens einem Alkanol und mindestens einem mehrwertigen Alkohol.

Die erfindungsgemäß verwendbaren Alkanole und/oder mehrwertigen Alkohole werden zweckmäßigerweise in einer Menge von 0,01 bis 5 Gew.-%, vorzugsweise 0,2 bis 1,6 Gew.-% und insbesondere 0,4 bis 0,8 Gew.-%, bezogen auf das Gewicht des lösungsmittelfreien Roh-MDI, eingesetzt.

Nach Abtrennung des überschüssigen Phosgens und des inerten Lösungsmittels können dem niedermolekulare Alkanole und/oder mehrwertige Alkohole und/oder Reaktionsprodukte, erhältlich aus diesen ein- und/oder mehrwertigen Alkoholen und Roh-MDI, enthaltenden Roh-MDI gegebenenfalls noch mindestens ein Antioxidans auf Phenolbasis, mindestens ein Arylphosphit oder eine Mischung dieser Stabilisatoren hinzugefügt werden. Sofern diese Stabilisatoren, die in Verbindung mit den erfindungsgemäß verwendeten niedermolekularen Alkanolen und/oder mehrwertigen Alkoholen eine zusätzliche Reduzierung der Iodfarbzahl bewirken können, Anwendung finden, werden sie zweckmäßigerweise in einer Menge von 0 bis maximal 5 Gew.-%, vorzugsweise von 0,01 bis 3 Gew.-% und insbesondere 0,1 bis 1,0 Gew.-%, bezogen auf das Gewicht des Roh-MDI, eingesetzt.

Als geeignete Antioxidantien auf Phenolbasis kommen beispielsweise in Betracht: Styrolisierte Phenole, das sind Phenole, die in 2- oder 4-Stellung oder in 2- und 4- und/oder 6-Stellung eine 1-Phenyl-ethylgruppe gebunden enthalten, Bis[2-hydroxy-5-methyl-3-tert.-butylphenyl]methan, 2,2-Bis-[4-hydroxy-phenyl]-propan, 4,4'-Dihydroxy-biphenyl, 3,3'-Dialkyl- bzw. 3,3',5,5'-Tetraalkyl-4,4'-dihydroxy-biphenyl, Bis-[4-hydroxy-2-methyl-5-tert.-butylphenyl]-sulfid, Hydrochinon, 4-Methoxy-, 4-tert.-Butoxy- oder 4-Benzyloxy-phenol, Gemische aus 4-Methoxy-2- bzw. -3-tert.-butylphenol, 2,5-Dihydroxy-1-tert.-butyl-benzol, 2,5-Dihydroxy-1,4-di-tert.-butyl-benzol, 4-Methoxy-2,6-di-tert.-butylphenol und vorzugsweise 2,6-Di-tert.-butyl-p-kresol.

Als Arylphosphite bewährt haben sich Tri-(alkylphenyl)-phosphite mit 1 bis 10 C-Atomen im Alkylrest, wie z.B. Tri-(methylphenyl)-, Tri-(ethylphenyl)-, Tri-(n-propylphenyl)-, Tri-(isopropylphenyl)-, Tri-(n-butylphenyl)-, Tri(sek.-butylphenyl)-, Tri-(tert.butylphenyl)-, Tri-(pentylphenyl)-, Tri-(hexylphenyl)-, Tri-(2-ethyl-hexylphenyl)-, Tri-(octylphenyl)-, Tri-(2-ethyl-octylphenyl)-, Tri-(decylphenyl)-phosphit und vorzugsweise Tri-(nonylphenyl)-phosphit, und insbesondere Triphenylphosphit.

Zur Herstellung der Roh-MDI mit reduzierter Iodfarbzahl nach dem erfindungsgemäßen Verfahren werden die entsprechenden Roh-MDA zweckmäßigerweise bei einer Temperatur im Bereich von 90 bis 220 °C, vorzugsweise von 120 bis 180 °C, bei erhöhtem Druck, z.B. bei 1 bis 10 bar, vorzugsweise 1 bis 3 bar oder insbesondere bei Normaldruck, phosgeniert. Die bei dem erfindungsgemäßen Verfahren angewandte Temperatur liegt über der Zersetzungstemperatur der als Zwischenprodukte durch die Reaktion von Roh-MDA mit Phosgen gebildeten Carbamidsäurechloride. Einer Erhöhung des Drucks sind nur technische und gegebenenfalls sicherheitstechnische Grenzen gesetzt, wobei jedoch mit einer größeren Druckerhöhung keine Ausbeutesteigerungen mehr verbunden sind.

Nach beendeter Phosgenierung wird der Reaktionsmischung, die aus mindestens einem inerten organischen Lösungsmittel, gelöstem Roh-MDI, überschüssigem Phosgen, Chlorwasserstoff sowie Nebenprodukten der Phosgenierung besteht, bei einer Temperatur von 20 bis 150 °C, vorzugsweise von 70 bis 120 °C und insbesondere 80 bis 110 °C das niedermolekulare Alkanol und/oder der mehrwertige Alkohol, insbesondere Glycerin einverleibt. Nach einer Verweilzeit von 0,1 bis 45 Minuten, vorzugsweise von 2 bis 25 Minuten bei einer Temperatur von 20 bis 150 °C, vorzugsweise von 70 bis 120 °C wird das überschüssige Phosgen bei Normaldruck und anschließend bei einer Temperatur von 30 bis 180 °C, vorzugsweise von 50 bis 150 °C das inerte organische Lösungsmittel oder Mischungen davon unter vermindertem Druck, z.B. bei einem Druck von 0,01 bis 100 mbar, vorzugsweise von 0,1 bis 50 mbar im wesentlichen vollständig, vorzugsweise durch Destillation, abgetrennt.

Den niedermolekulare Alkanole und/oder mehrwertige Alkohole und/oder Reaktionsprodukte dieser Alkohole mit Roh-MDI enthaltenden Roh-MDI's können nunmehr, sofern dies zweckdienlich erscheint, mindestens ein Antioxidans auf Phenolbasis und/oder mindestens ein Arylphosphit in einer wirksamen Menge hinzugefügt werden. Danach werden die auf diese Weise behandelten Roh-MDI zur Entchlorierung auf eine Temperatur von 100 bis 250 °C, vorzugsweise von 140 bis 200 °C erhitzt und bei dieser Temperatur unter einem Druck von 0,01 bis 100 mbar, vorzugsweise von 0,1 bis 20 mbar mindestens 5 Minuten und insbesondere 5 bis 45 Minuten, behandelt. Nach der Abkühlung auf 60 °C wird das Roh-MDI der Zwischenlagerung zugeführt und dort weiter abkühlen gelassen.

Die nach dem erfindungsgemäßen Verfahren hergestellten Roh-MDI besitzen eine deutlich reduzierte Iodfarbzahl, üblicherweise von maximal 60 und finden Verwendung zur Herstellung von kompakten oder geschäumten Polyisocyanat-polyadditionsprodukten, vorzugsweise flexiblen, halbharten oder harten Urethan- oder Urethan- und Isocyanuratgruppen enthaltenden Schaumstoffen, die eine deutlich hellere Farbe aufweisen.

Beispiele 1 bis 13 und Vergleichsbeispiele I bis V

Zu einer Reaktionsmischung, die bezogen auf 100 Gew.-Teile, bestand aus
80 Gew.-Teilen Monochlorbenzol als Lösungsmittel,
10 Gew.-Teilen überschüssiges Phosgen und
10 Gew.-Teilen Roh-MDI, das seinerseits enthielt:
    50 Gew.-% 4,4'-MDI,
    4 Gew.-% 2,4'-MDI,
    0,04 Gew.-% 2,2'-MDI und
    45,96 Gew.-% Homologe mit mehr als zwei Isocyanatgruppen, bezogen auf das Roh-MDI-Gewicht,
fügte man bei einer Temperatur von 105 °C die Alkanole oder mehrwertigen Alkohole oder die Vergleichssubstanzen.

Die Reaktionsmischung wurde danach in ungefähr 20 Minuten auf 140 °C erwärmt und unter Normaldruck das überschüssige Phosgen innerhalb 20 Minuten mit Hilfe eines Rotationsverdampfers abdestilliert.

Die Reaktionsmischung ließ man anschließend in ungefähr 10 Minuten auf 100 bis 120 °C abkühlen und destillierte in diesem Temperaturbereich unter vermindertem Druck (50 bis 10 mbar) innerhalb von ungefähr 15 Minuten das Monochlorbenzol im wesentlichen vollständig ab.

Dem erhaltenen Alkanol und/oder mehrwertige Alkohole und/oder Reaktionsprodukte dieser Alkohole mit Roh-MDI enthaltenden Roh-MDI wurden nunmehr gegebenenfalls die Antioxidantien auf Phenolbasis oder das Arylphosphit einverleibt und danach das Alkanol und/oder mehrwertige Alkohole und/oder Reaktionsprodukte dieser Alkohole mit Roh-MDI und gegebenenfalls die Stabilisatoren enthaltende Roh-MDI bei 180 °C oder 210 °C und 10 mbar 30 Minuten lang entchloriert.

Die eingesetzten Alkanole und mehrwertigen Alkohole sowie die Vergleichssubstanzen und gegebenenfalls Stabilisatoren und ihre Mengen sowie die an den erhaltenen Roh-MDI gemessenen Iodfarbzahlen sind in der folgenden Tabelle zusammengefaßt.

5

Tabelle

| Vergleichs-beispiele | | Zusatz nach beendeter Phosgenierung | | Zusatz nach Abdestillation des Lösungsmittel | | Iodfarbzahl nach der Entchlorierung bei 180°C bzw. 210°C | |
|---|---|---|---|---|---|---|---|
| | | Art | Menge [%]* | Art | Menge [%]* | | |
| | Roh-MDI | - | - | - | - | 100 | 100 |
| I | Roh-MDI | Wasser | 1,0 | - | - | 12 | 15 |
| II | Roh-MDI | Wasser | 0,5 | - | - | 20 | 30 |
| III | Roh-MDI | Wasser | 0,1 | - | - | 20 | 30 |
| IV | Roh-MDI | Wasser | 0,05 | - | - | 20 | 30 |
| V | Roh-MDI | Wasser | 0,02 | - | - | 30 | 35 |
| Beispiele | | Alkanole oder mehrwertige Alkohole | | Stabilisator | | | |
| 1 | Roh-MDI | Methanol | 0,5 | - | - | 40 | 60 |
| 2 | Roh-MDI | Butanol | 0,5 | - | - | 50 | 60 |
| 3 | Roh-MDI | Ethylenglykol | 0,02 | - | - | 30 | 40 |
| 4 | Roh-MDI | Ethylenglykol | 0,5 | - | - | 20 | 35 |
| 5 | Roh-MDI | Ethylenglykol | 0,5 | Di-tert.butyl-kresol | 0,5 | 18 | 35 |
| 6 | Roh-MDI | Ethylenglykol | 0,5 | Triphenylphosphit | 0,5 | 18 | 15 |
| 7 | Roh-MDI | Glycerin | 0,1 | - | - | 30 | 35 |
| 8 | Roh-MDI | Glycerin | 0,4 | - | - | 12 | 20 |
| 9 | Roh-MDI | Glycerin | 0,7 | - | - | 9 | 20 |

EP 0 445 602 B1

Tabelle - Forts.

| Beispiele | | Alkanole oder mehrwertige Alkohole | | Stabilisator | | Iodfarbzahl nach der Entchlorierung bei 180°C bzw. 210°C | |
| --- | --- | --- | --- | --- | --- | --- | --- |
| | | Art | Menge [%]* | Art | Menge [%]* | | |
| 10 | Roh-MDI | Glycerin | 1,0 | - | - | 7 | 18 |
| 11 | Roh-MDI | Glycerin | 1,5 | - | - | 6 | 18 |
| 12 | Roh-MDI | Glycerin | 0,5 | Di-tert.butyl-kresol | 0,5 | 8 | 18 |
| 13 | Roh-MDI | Glycerin | 0,5 | Triphenylphosphit | 0,5 | 8 | 20 |

* bezogen auf Roh-MDI

**Patentansprüche**

1. Verfahren zur Herstellung von Mischungen aus Diphenylmethan-diisocyanaten und Polyphenyl-polymethylen-polyisocyanaten (Roh-MDI) mit einer Iodfarbzahl von kleiner als 60 durch Umsetzung der entsprechenden Mischungen aus

EP 0 445 602 B1

Diphenylmethan-diaminen und Polyphenyl-polymethylen-polyaminen mit Phosgen in Gegenwart mindestens eines inerten organischen Lösungsmittels bei erhöhter Temperatur,
nach beendeter Phosgenierung Abtrennung des überschüssigen Phosgens und Lösungsmittels und thermische Behandlung des Reaktionsprodukts,
dadurch gekennzeichnet, daß man der Reaktionsmischung nach beendeter Phosgenierung Alkanole mit 1 bis 10 Kohlenstoffatomen oder mehrwertige Alkohole oder Mischungen davon in einer Menge von 0,01 bis 5 Gew.-%, bezogen auf das Gewicht des lösungsmittelfreien Roh-MDI, einverleibt.

2. Verfahren nach Anspruch 1, dadurch gekennzeichnet, daß die mehrwertigen Alkohole 2- bis 8-wertig sind und ein Molekulargewicht von 60 bis 350 besitzen.

3. Verfahren nach Anspruch 1 dadurch gekennzeichnet, daß der mehrwertige Alkohol aus Glycerin besteht.

4. Verfahren nach einem der Ansprüche 1 bis 3, dadurch gekennzeichnet, daß die erhaltenen Mischungen aus Diphenylmethan-diisocyanaten und Polyphenyl-polymethylen-polyisocyanaten einen Diphenylmethan-diisocyanat-Isomerengehalt von 30 bis 90 Gew.-%, einen NCO-Gehalt von 31±2 Gew.-%, jeweils bezogen auf das Gesamtgewicht, eine Viskosität von maximal 2000 m•Pa•s bei 23°C und eine Iodfarbzahl von kleiner als 60 besitzen.

5. Verfahren nach einem der Ansprüche 1 bis 4, dadurch gekennzeichnet, daß man der niedermolekulare Alkanole und/oder mehrwertige Alkohole enthaltenden Mischung aus Diphenylmethan-diisocyanaten und Polyphenyl-polymethylen-polyisocyanaten nach der Abtrennung des überschüssigen Phosgens und des inerten organischen Lösungsmittels und vor der thermischen Behandlung des Reaktionsprodukts mindestens ein Antioxidans auf Phenolbasis und/oder mindestens ein Arylphosphit einverleibt.

6. Verfahren nach einem der Ansprüche 1 bis 4, dadurch gekennzeichnet, daß man der niedermolekulare Alkanole und/oder mehrwertige Alkohole enthaltenden Mischung aus Diphenylmethan-diisocyanaten und Polyphenyl-polymethylen-polyisocyanaten nach der Abtrennung des überschüssigen Phosgens und des inerten organischen Lösungsmittels und vor der thermischen Behandlung des Reaktionsprodukts mindestens ein Anti-oxidans auf Phenolbasis in einer Menge von maximal 5 Gew.-% und/oder mindestens ein Arylphosphit in einer Menge von maximal 5 Gew.-%, jeweils bezogen auf das Gewicht der Mischung aus Diphenylmethan-diisocyanaten und Polyphenyl-polymethylen-polyisocyanaten, einverleibt.

7. Verfahren nach einem der Ansprüche 5 oder 6, dadurch gekennzeichnet, daß das Antioxidans auf Phenolbasis aus Di-tert.butyl-p-kresol und das Arylphosphit aus Triphenylphosphit bestehen.

8. Verfahren nach Anspruch 1, dadurch gekennzeichnet, daß man der Reaktionsmischung nach beendeter Phosgenierung 0,01 bis 5 Gew.-% Glycerin, bezogen auf das Gewicht der Mischung aus Diphenylmethan-diisocyanaten und Polyphenyl-polymethylen-polyisocyanaten in der Reaktionsmischung, einverleibt, danach das überschüssige Phosgen und das inerte organische Lösungsmittel abdestilliert, der Reaktionsmischung 0 bis 5 Gew.-% Di-tert.-butyl-p-kresol und/oder Triphenylphosphit, bezogen auf das Gewicht der Mischung aus Diphenylmethan-diisocyanaten und Polyphenyl-polymethylen-polyisocyanaten, hinzufügt und danach das Reaktionsprodukt thermisch behandelt.

Claims

1. A process for the preparation of a mixture of diphenylmethane diisocyanates and polyphenyl-polymethylene polyisocyanates (crude MDI) having an iodine color number of less than 60 by reacting the corresponding mixture of a diphenylmethanediamine and a polyphenyl-polymethylenepolyamine with phosgene in the presence of one or more inert organic solvents at elevated temperatures, separating off the excess phosgene and solvent after the end of the phosgenation and subjecting the reaction product to a thermal treatment, wherein an alkanol of 1 to 10 carbon atoms or a polyhydric alcohol or a mixture thereof is incorporated into the reaction mixture in an amount of from 0.01 to 5% by weight, based on the weight of the solvent-free crude MDI, after the end of the phosgenation.

8

**2.** A process as claimed in claim 1, wherein the polyhydric alcohol is dihydric to octahydric and has a molecular weight of from 60 to 350.

**3.** A process as claimed in claim 1, wherein the polyhydric alcohol is glycerol.

**4.** A process as claimed in claim 1 or 2 or 3, wherein the resulting mixture of diphenylmethane diisocyanates and polyphenylpolymethylene polyisocyanates has a diphenylmethane diisocyanate isomer content of from 30 to 90% by weight, an NCO content of 31 ± 2% by weight, based in each case on the total weight, a viscosity of not more than 2,000 mPa.s at 23°C and an iodine color number of less than 60.

**5.** A process as claimed in claim 1 or 2 or 3 or 4, wherein one or more phenol-based antioxidants and/or one or more aryl phosphites are incorporated in the mixture of diphenylmethane diisocyanates and polyphenylpolymethylene polyisocyanates, containing low molecular weight alkanols and/or polyhydric alcohols, after the excess phosgene and the inert organic solvent have been separated off and before the reaction product has been subjected to thermal treatment.

**6.** A process as claimed in claim 1 or 2 or 3 or 4, wherein one or more phenol-based antioxidants in an amount of not more than 5% by weight and/or one or more aryl phosphites in an amount of not more than 5% by weight, based in each case on the weight of the mixture of diphenylmethane diisocyanates and polyphenylpolymethylene polyisocyanates, are incorporated into the mixture of diphenylmethane diisocyanates and polyphenyl-polymethylene polyisocyanates, containing low molecular weight alkanols and/or polyhydric alcohols, after the excess phosgene and the inert organic solvent have been separated off and before the reaction product has been subjected to a thermal treatment.

**7.** A process as claimed in claim 5 or 6, wherein the phenol-based antioxidant consists of di-tert-butyl-p-cresol and the aryl phosphite consists of triphenyl phosphite.

**8.** A process as claimed in claim 1, wherein from 0.01 to 5% by weight, based on the weight of the mixture of diphenylmethane diisocyanates and polyphenylpolymethylene polyisocyanates in the reaction mixture, of glycerol are incorporated into the reaction mixture after the end of the phosgenation, after which the excess phosgene and the inert organic solvent are distilled off, from 0 to 5% by weight, based on the weight of the mixture of diphenylmethane diisocyanates and polyphenyl-polymethylene polyisocyanates, of di-tert-butyl-p-cresol and/or triphenyl phosphite are added to the reaction mixture, and the reaction product is then subjected to a thermal treatment.

**Revendications**

**1.** Procédé pour la préparation de mélanges de diphénylméthane-diisocyanates et de polyphényl-polyméthylène-polyisocyanates (MDI brut) ayant un indice de couleur à l'iode inférieur à 60, par réaction des mélanges correspondants de diphénylméthane-diamines et polyphényl-polyméthylène-polyamines avec le phosgène en présence d'au moins un solvant organique inerte à chaud, séparation de l'excès de phosgène et du solvant après phosgénation, et traitement thermique du produit de réaction, caractérisé en ce que, après phosgénation, on incorpore au mélange de réaction des alcanols en C1-C10 ou des alcools polyvalents ou leurs mélanges en quantité de 0,01 à 5 % du poids du MDI brut exempt de solvant.

**2.** Procédé selon la revendication 1, caractérisé en ce que les alcools polyvalents ont une valence de 2 à 8 et un poids moléculaire de 60 à 350.

**3.** Procédé selon la revendication 1, caractérisé en ce que l'alcool polyvalent est du glycèrol.

**4.** Procédé selon l'une des revendications 1 à 3, caractérisé en ce que les mélanges de diphénylméthane-diisocyanates et de polyphényl-polyméthylène-polyisocyanates ainsi obtenus ont une teneur en isomères du diphénylméthane-diisocyanate de 30 à 90 % en poids, une teneur en NCO de 31 ± 2 % en poids, dans les deux cas par rapport au poids total, une viscosité maximale de 2 000 mPa.s à 23°C et un indice de couleur à l'iode inférieur à 60.

**5.** Procédé selon l'une des revendications 1 à 4, caractérisé en ce que, après séparation de l'excès de phosgène et du solvant organique inerte et avant le traitement thermique du produit de réaction, on incorpore un antioxydant à base phénolique et/ou au moins un phosphite d'aryle au mélange de diphénylméthane-diisocyanates et de polyphényl-polyméthylène-polyisocyanates contenant les alcools à bas poids moléculaire et/ou alcools polyvalents.

**6.** Procédé selon l'une des revendications 1 à 4, caractérisé en ce que, après séparation de l'excès de phosgène et du solvant organique inerte et avant le traitement thermique du produit de réaction, on incorpore au moins un antioxydant à base phénolique en quantité de 5 % en poids au maximum et/ou au moins un phosphite d'aryle en quantité de 5 % en poids au maximum au mélange de diphénylméthane-diisocyanates et de polyphényl-polyméthylène-polyisocyanates contenant les alcanols à bas poids moléculaire et/ou alcools polyvalents, dans les deux cas par rapport au poids du mélange de diphénylméthane-diisocyanates et de polyphényl-polyméthylène-polyisocyanates.

**7.** Procédé selon l'une des revendications 5 ou 6, caractérisé en ce que l'antioxydant à base phénolique est le di-tert-butyl-p-crésol et le phosphite d'aryle est le phosphite de triphényle.

**8.** Procédé selon la revendication 1, caractérisé en ce que, après phosgénation, on incorpore au mélange de réaction de 0,01 à 5 % en poids de glycérol, par rapport au poids du mélange de diphénylméthane-diisocyanates et de polyphényl-polyméthylène-polyisocyanates contenu dans le mélange de réaction puis on distille l'excès de phosgène et le solvant organique inerte, on ajoute au mélange de réaction de 0 à 5 % en poids de di-tert-butyl-p-crésol et/ou de phosphite de triphényle, par rapport au poids du mélange de diphénylméthane-diisocyanates et de polyphényl-polyméthylène-polyisocyanates, puis on soumet le produit de réaction au traitement thermique.